# EUROPEAN PATENT APPLICATION

(11) **EP 1 092 712 A1**
(43) Date of publication of application: **18.04.2001**
(21) Application number: 99500185.6
(22) Date of filing: 13.10.1999
(51) Int. Cl.: C07D 263/44, C07D 263/46, C07D 233/74, C07D 233/76, C07D 233/86, C07C 69/96, C07C 271/22, C07C 271/28, C07C 329/04, C07C 333/04

(54) **Process for preparing fungicidal oxazolidinones and imidazolinones**

(71) Applicant: E.I. DU PONT DE NEMOURS AND COMPANY, Wilmington Delaware 19898 (US)
(72) Inventor: Armesto Abella, Nuria, 33210 Gijon (Asturias) (ES); Lavandera Garcia, Ivan, 33008 Ovieda (Asturias) (ES); Ferrero Fuertes, Miguel, 33013 Oviedo (Asturias) (ES); Gotor-Fernandez, Vicente, 33008 Ovieda (Asturias) (ES); Bellettini, Arturo G., 33394 Cabuenes (Asturias) (ES); Sternberg, Jeffrey Arthur, Wilmington, Delaware 19808 (US); Fernandez Gonzalez, Susana, 33013 Oviedo (Asturias) (ES); Gotor Santamaria, Vicente, 33008 Oviedo (Asturias) (ES)
(74) Representative: Carpintero Lopez, Francisco

(57) **Abstract**

The present invention pertains to a process for preparing compounds of Formula I (useful as fungicides and/or intermediates for producing fungicides) 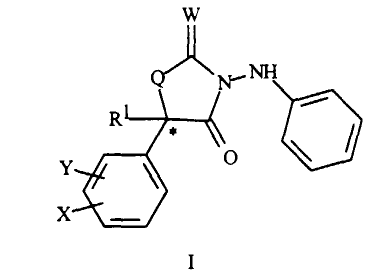 wherein Q is O or NH; W is O or S; X is H, halogen, C₁-C₄ alkyl, C₁-C₄ haloalkyl or OR⁴; Y is H or halogen; R¹ is C₁-C₄ alkyl; R⁴ is C₁ -C₄ alkyl, C₁ -C₄ haloalkyl or optionally substituted phenyl; and * represents the location of the chiral center; and important intermediates of Formula II used in that process.

The process of this invention comprises treating a compound of Formula II (that is racemic, enantiomerically enriched, or enantiomerically pure at chiral center *), or esters thereof, 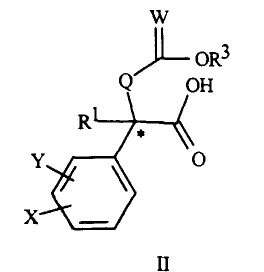 wherein OR³ is an oxygen-linked coupling agent (and Q, W, X, R¹, R⁴ and * are as previously defined); with phenyl hydrazine in the presence of an acidic catalyst (and preferrably a basic catalyst).

## Description

### BACKGROUND OF THE INVENTION

This invention pertains to a novel process for the preparation of fungicidal oxazolidinones and intermediates to imidazolinone fungicides, and important intermediates used in that process.

Fungicides that effectively control plant diseases are in constant demand by growers. Plant diseases are highly destructive, difficult to control and quickly develop resistance to commercial fungicides. U.S. 4,957,933 and *Synthesis,* **1981,** 38-40 disclose the preparation of 2,4-oxazolidinedione fungicides by treatment of *N*-hydroxy-2-hydroxyhydroxamic acids with 1,1'-carbonyldiimidazole to form dioxazinediones, and subsequent treatment of the dioxazinediones with phenylhydrazine. WO 90/12791 discloses the preparation of 2,4-oxazolidinediones from 2-thioxooxazolidin-4-ones by desulfurization. WO94/11359 discloses the preparation of 2,4-oxazolidinedione fungicides by treatment of 2-hydroxycarboxylic esters with 1,1'-carbonyldiimidazole or 1,1'-carbonylditriazole and subsequent treatment of the intermediate with phenylhydrazine. However, there is a need for a more efficient process. The present invention provides a new efficient process for the preparation of 2,4-oxazolidinediones.

EP-0551,048; EP-0629,616; W093/24467 and W096/03044 disclose certain imidazolinone fungicides and processes to prepare them. The process of the present invention is not disclosed. However, there is a need for a more efficient process. The present invention provides a new efficient process for the preparation of a key intermediate for the preparation of imidazolinone fungicides.

### SUMMARY OF THE INVENTION

The present invention pertains to a process for preparing compounds of Formula I useful as fungicides and/or intermediates for producing fungicides. 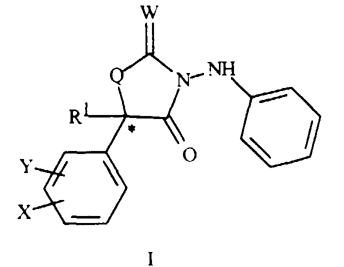 wherein Q is O or NH; W is O or S; X is H, halogen, C₁-C₄ alkyl, C₁-C₄ haloalkyl or OR⁴; Y is H or halogen; R¹ is C₁-C₄ alkyl; R⁴ is C₁-C₄ alkyl, C₁-C₄ haloalkyl or optionally substituted phenyl; and * represents the location of a chiral center. Compounds of Formula I may be racemic, enantiomerically enriched, or enantiomerically pure at chiral center *.

The process of this invention comprises treating a compound of Formula II (that is racemic, enantiomerically enriched, or enantiomerically pure at chiral center *), or esters thereof, 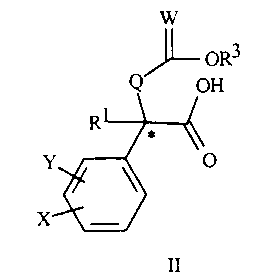 wherein OR³ is an oxygen-linked coupling moiety (and Q, W, X, Y, R¹ and * are as previously defined); with phenyl hydrazine in the presence of an acidic catalyst (and preferrably a basic catalyst).

This invention also includes compounds of Formula II and esters thereof, 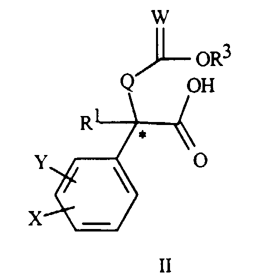 wherein Q is O or NH; W is O or S; X is H, halogen, C₁-C₄ alkyl, C₁-C₄ haloalkyl or OR⁴; Y is H or halogen; R¹ is C₁-C₄ alkyl; OR³ is an oxygen-linked coupling moiety; and R⁴ is C₁-C₄ alkyl, C₁-C₄ haloalkyl or optionally substituted phenyl; provided that (a) when Q and W are both O and X and Y are both H, then R³ is other than CH₃; and (b) when Q is NH, then (i) R³ is other than ethyl, benzyl and benzyl substituted with -N=N-phenyl, and (ii) the esters of Formula II are other than C₁-C₄ alkyl esters, C₃-C₄ alkenyl esters, C₃-C₆ cycloalkyl esters and benzyl esters.

### DETAILED DESCRIPTION OF THE INVENTION

Compounds of Formula I can be prepared from precursors of compounds of Formula II using a multistep process comprising (A) treating a compound of Formula III (that is racemic, enantiomerically enriched, or enantiomerically pure at chiral center *), or esters thereof, 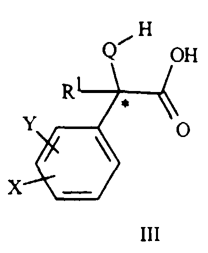 wherein Q, X, Y, and R¹ are as previously defined; with a chloroformate of Formula IV 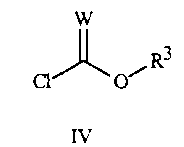 wherein W is O or S and OR³ is an oxygen-linked coupling moiety; in the presence of a base; to form a compound of Formula II as previously defined; and
(B) treating the compound of Formula II as indicated above.
This process is particularly of note when Q and W are both O.

The process can be carried out by
(1) conducting Steps A and B sequentially without isolating compounds of Formula II or
(2) conducting Step A, isolating compounds of Formula II, and conducting Step B in a separate reaction.

One skilled in the art will recognize that some compounds of Formula I can exist in one or more tautomeric forms. For example, a compound of Formula I wherein Q is NH may exist as tautomer Ia or Ib, or both Ia and Ib. The present invention comprises all tautomeric forms of compounds of Formula I.

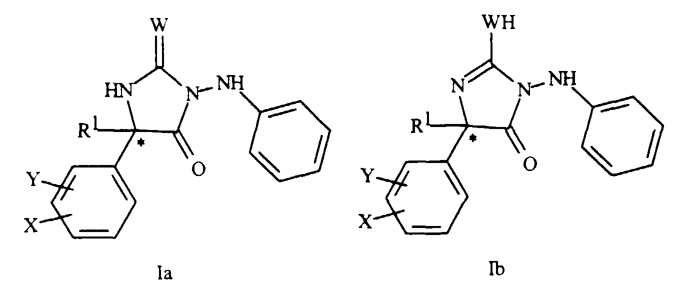

Esters of Formulae II and III include compounds of Formulae IIa and IIIa, respectively, wherein R² is C₁-C₄ alkyl; C₁-C₄ haloalkyl; or phenyl, benzyl or phenethyl, each optionally substituted on the aromatic ring. Examples of optional substituents include halogen, nitro, C₁-C₄ alkyl, C₁-C₄ haloalkyl, C₁-C₄ alkoxy, C₁-C₄ haloalkoxy and cyano.

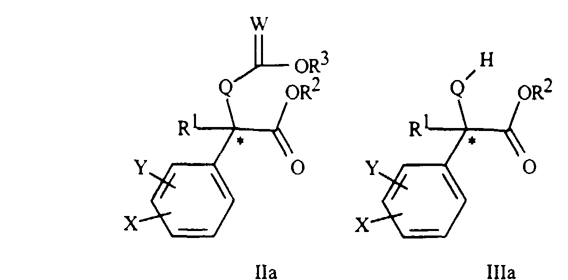

In compounds of Formula II, OR³ is an oxygen-linked coupling moiety. This is a moiety linked to the carbonyl through the oxygen atom of OR³ and which activates transacylation with phenylhydrazine. Examples of OR³ include C₁-C₄ alkoxy; C₁-C₄ haloalkoxy; and phenoxy, benzyloxy and phenethyloxy, each optionally substituted on the aromatic ring. Examples of optional substituents include halogen, nitro, C₁-C₄ alkyl, C₁-C₄ haloalkyl, C₁-C₄ alkoxy, C₁-C₄ haloalkoxy and cyano. Further examples of OR³ include 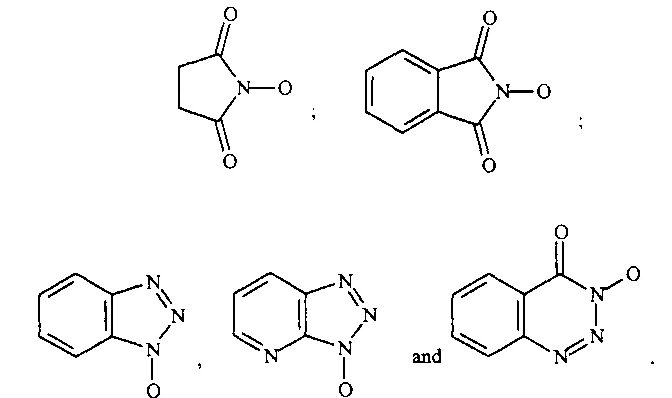 These moieties are well-known in the art for their ability to facilitate transacylation reactions (for a review of their use in peptide couplings, see Klausner, Y. S. and Bodansky, M., *Synthesis,* **1972,** 453-63; Carpino, L. A., *J. Am. Chem. Soc.,* **1993,** 115, 4397-8).

When R⁴ is optionally substituted phenyl (see the definition of X), examples of optional substituents include halogen, nitro, C₁-C₄ alkyl, C₁-C₄ haloalkyl, C₁-C₄ alkoxy, C₁-C₄ haloalkoxy and cyano.

Of note are compounds of Formula IIb, which, when treated with phenyl hydrazine as described above, can provide compounds of Formula I wherein Q and W are both O with the concurrent release of a compound of Formula IIIb.

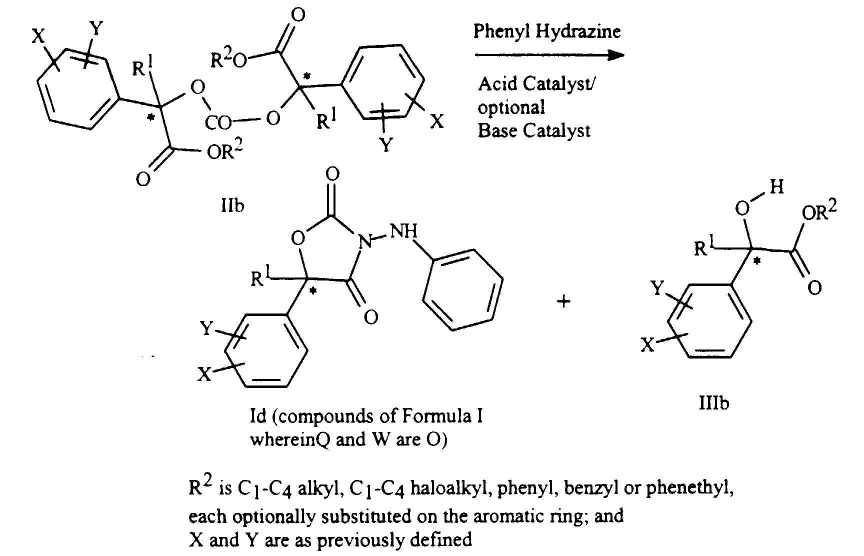

The compounds of Formulae I, II and III can exist as enantiomers. One skilled in the art will appreciate how to separate said enantiomers. Accordingly, the present invention comprises processess for preparing the racemic mixtures, the individual enantiomers or enantiomerically enriched mixtures of compounds of Formulae I or II, or agriculturally suitable salts thereof. Although one enantiomer may have superior fungicidal activity for a given compound of Formula I, the other enantiomer is not devoid of activity nor does it interfere with the activity of the more potent enantiomer. The enantiomer of Formula I which provides greater fungicidal activity, and is therefore preferred, is that illustrated as Formula Ic. 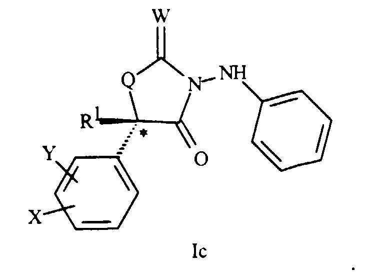 In the present invention, preparing compounds of Formula I enantiomerically pure or enantiomerically enriched in the enantiomer of Formula Ic uses compounds of Formula II that are enantiomerically pure or enantiomerically enriched in the enantiomer of Formula IIc, or esters thereof,

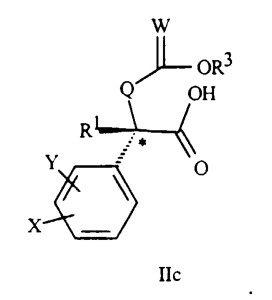

In the above recitations, the term "alkyl", used either alone or in compound words such as "haloalkyl" denotes straight-chain or branched alkyl; e.g., methyl, ethyl, *n*-propyl, *i*-propyl, or the different butyl isomers. The term "C₁-C₂ alkyl" denotes methyl or ethyl. "Alkoxy" includes, for example, methoxy, ethoxy, n-propyloxy, isopropyloxy and the different butoxy isomers. The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different. Examples of "haloalkyl" include F₃C, ClCH₂, CF₃CH₂ and CF₃CCl₂. The term "phenoxy" denotes OC₆H₅, "benzyloxy" denotes OCH₂C₆H₅ and "phenethyloxy" denotes either 1-phenethyloxy (OCH(CH₃)C₆H₅) or 2-phenethyloxy (OCH₂CH₂C₆H₅). The term "aromatic ring" denotes fully unsaturated carbocycles in which the ring is aromatic (where aromatic indicates that the Hückel rule is satisfied for the ring system). The term "optionally substituted" in regard to aromatic rings means that one or more of the hydrogens attached to the aromatic ring may be replaced by a substituent. The total number of carbon atoms in a substituent group is indicated by the "Cᵢ-Cⱼ" prefix where i and j are numbers from 1 to 6. The term "nitro" denotes NO₂.

Preferred processes for reasons of cost and/or ease of synthesis are:
Preferred 1.
   The process for preparing a compound of Formula I that is enriched in the stereoisomer Ic 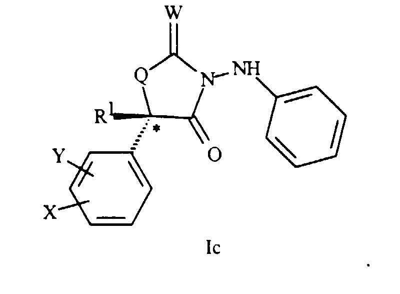
Preferred 2.
   The process for preparing a compound of Formula I wherein the acidic catalyst is selected from carboxylic acids, Lewis acids, trialkylammonium halides and pyridine carboxylic acids; and wherein a basic catalyst selected from tertiary amines and heteroaryl amines is also present.
Preferred 3.
   The process of Preferred 2 wherein an ester of Formula IIa 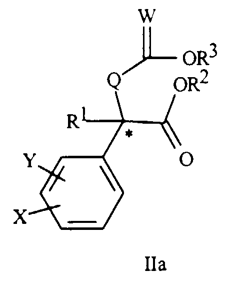 wherein
   R² is C₁-C₄ alkyl; C₁-C₄ haloalkyl; or phenyl, benzyl or phenethyl, each optionally substituted on the aromatic ring with one or more substituents selected from halogen, nitro, C₁-C₄ alkyl, C₁-C₄ haloalkyl, C₁-C₄ alkoxy, C₁-C₄ haloalkoxy and cyano; and
   R³ is phenyl optionally substituted with one or more substituents selected from halogen, nitro, C₁-C₄ alkyl, C₁-C₄ haloalkyl, C₁-C₄ alkoxy, C₁-C₄ haloalkoxy and cyano;
   is treated with phenyl hydrazine; the acidic catalyst is selected from the group consisting of acetic acid, pivalic acid, benzoic acid and nicotinic acid; the basic catalyst is *N,N-*dimethylaminopyridine; the reaction is carried out in a solvent selected from the group anisole, xylene, tetrahydrofuran and acetonitrile; and the reaction temperature is in the range from 50 to 100° C.
Preferred 4.
   The process for preparing a compound of Formula I wherein Q is O; W is O; X is H, halogen or 4-phenoxy; Y is H; and R¹ is methyl.
Preferred 5.
   The process for preparing a compound of Formula I wherein Q is NH; W is S; X is H, halogen, C₁-C₄ alkyl, C₁-C₄ haloalkyl or OR⁴; Y is H or halogen; R¹ is methyl; and R⁴ is C₁-C₄ alkyl, C₁-C₄ haloalkyl or optionally substituted phenyl.
Preferred 6.
   The process for preparing a compound of Formula I wherein the compound of Formula II is prepared by treating a compound of Formula IIIa, or an ester thereof, 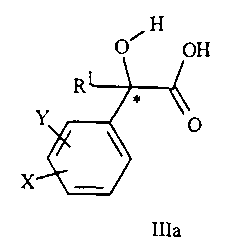 with a chloroformate of Formula IVa 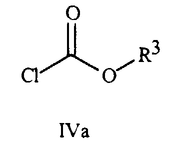 wherein R³ is C₁-C₄ alkyl or phenyl optionally substituted with one or more substituents selected from halogen, nitro, C₁-C₄ alkyl, C₁-C₄ haloalkyl, C₁-C₄ alkoxy, C₁-C₄ haloalkoxy and cyano; in the presence of a base in an inert solvent at a temperature from 0 to 150° C. Preferred compounds of this invention for reasons of better activity, cost and/or ease of synthesis are:
Preferred 7.
   Compounds of Formula II that are enriched in the stereoisomer IIc, or esters thereof, 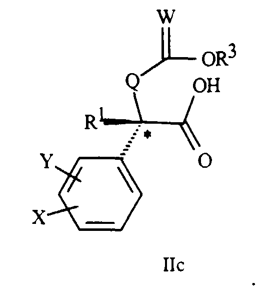
Preferred 8.
   Esters of Formula II of Formula IIa 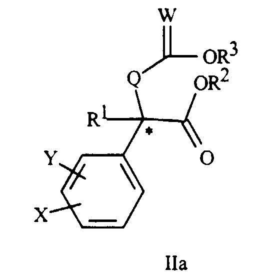 wherein
   R² is C₁-C₄ alkyl; C₁-C₄ haloalkyl; or phenyl, benzyl or phenethyl, each optionally substituted on the aromatic ring with one or more substituents selected from halogen, nitro, C₁-C₄ alkyl, C₁-C₄ haloalkyl, C₁-C₄ alkoxy, C₁-C₄ haloalkoxy and cyano; and
   R³ is phenyl optionally substituted with one or more substituents selected from halogen, nitro, C₁-C₄ alkyl, C₁-C₄ haloalkyl, C₁-C₄ alkoxy, C₁-C₄ haloalkoxy and cyano.
Preferred 9.
   Compounds of Formula IIa wherein Q is O; W is O; X is H or 4-phenoxy; Y is H;
   R¹ is methyl; R² is C₁-C₂ alkyl; and R³ is phenyl.

The present invention comprises a process for the preparation of 2,4-oxazolidinediones and imidazolinones as outlined in Scheme 1.

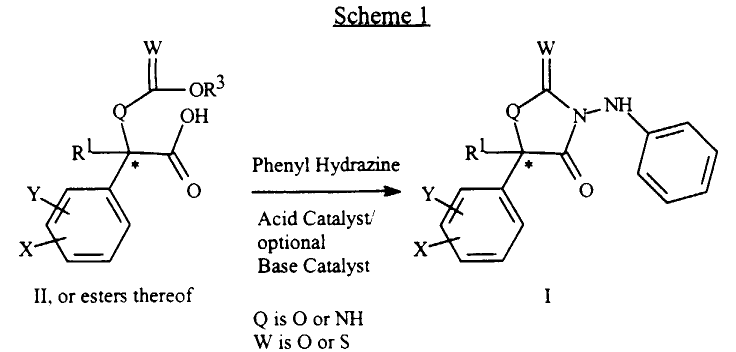

For the conversion of acids or esters of Formula II to compounds of Formula I, suitable solvents include inert organic solvents or a biphasic mixture of inert organic solvents and water. Preferred solvents are methylene chloride, chloroform, carbon tetrachloride, anisole, acetonitrile, hexanes, tetrahydrofuran, *tert*-butyl methyl ether, dioxanes, chlorobenzene, *o*-dichlorobenzene (ODCB), toluene, xylenes, and suitable combinations thereof. More preferred solvents are selected from the group consisting of anisole, acetonitrile, tetrahydrofuran and xylenes. The reaction temperatures can range, for example, from about 0°C to about 150°C. Preferred temperatures are from about 50°C to about 100°C. Typical reaction pressures are from about 1.0 x 10⁵ to about 5.1 x 10⁵ Pascals. The preferred pressure is about 1 x 10⁵ Pascals. The reaction times are typically 1 to 24 hours, preferably 3 to 6 hours. The acids suitable for catalyzing the reaction are selected from the group consisting of alkyl and aryl carboxylic acids, Lewis acids, trialkylammonium halides, pyridine carboxylic acids and combinations thereof. The acids may also be contained in a solid phase, such as in polymeric ion-exchange resins. The preferred acids are acetic acid, pivalic acid, benzoic acid or nicotinic acid. The most preferred acid is acetic acid. In biphasic solvent systems or heterogeneous systems, suitable phase-transfer catalysts may also be employed. Such phase-transfer catalysts include tetraalkylammonium salts. Typical mole ratios of phenylhydrazine to the compound of Formula II is from about 2:1 to 1:1. The preferred ratio is from about 1.6:1 to 1.1:1. An additional basic catalyst can also be used to accelerate the reaction. Suitable bases for this reaction include tertiary amines such as trialkylamine or heteroarylamines such as imidazole, pyridine, picoline or other substituted pyridine, or mixtures thereof. The bases may also be contained in a solid phase, such as in polymeric ion-exchange resins. The most preferred base is *N,N*-dimethylaminopyridine.

Compounds of Formula II, or esters thereof, can be prepared as illustrated in Scheme 2 by treating compounds of Formula III, or esters thereof, with (thio)chloroformates of Formula IV.

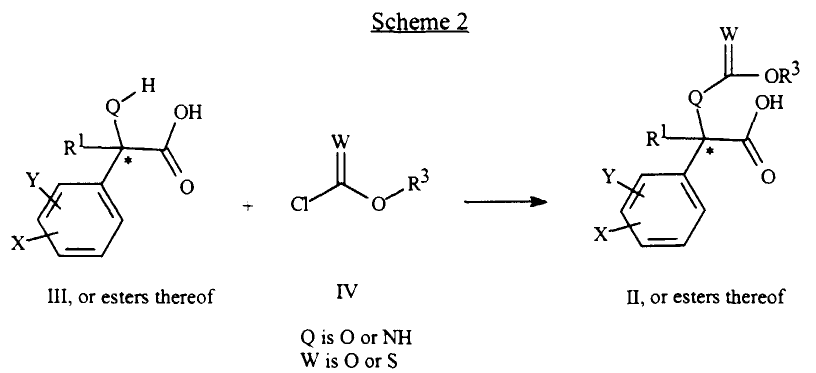

Compounds of Formula IIb can be prepared as illustrated in Scheme 2a by treating two equivalents of compounds of Formula IIIb with one equivalent of phosgene or phosgene equivalents such as diphosgene (trichloromethyl chloroformate) or triphosgene (bis(trichloromethyl)-carbonate).

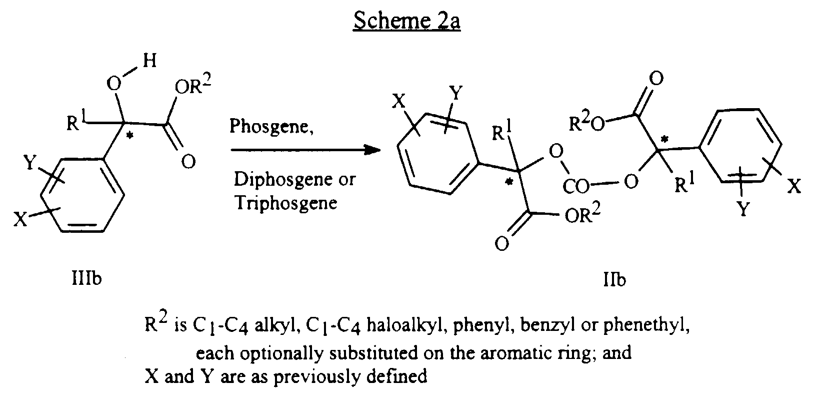

For the conversion of compounds of Formula III to compounds of Formula II, and compounds of Formula IIIb to compounds of Formula IIb, suitable solvents include inert organic solvents. Preferred solvents are methylene chloride, chloroform, carbon tetrachloride, anisole, acetonitrile, hexanes, heptanes, tetrahydrofuran, tert-butyl methyl ether, dioxanes, chlorobenzene, *o*-dichlorobenzene (ODCB), toluene, xylenes, and suitable combinations thereof. The reaction temperatures are typically from about 0°C to about 75°C. Preferred temperatures are from about 10°C to about 50°C. Reaction pressures are typically from about 1.0 x 10⁵ to about 5.1 x 10⁵ Pascals. The preferred pressure is about 1 x 10⁵ Pascals. The reaction times are typically 1 to 24 hours, preferably 2 to 6 hours. The chloroformate of Formula IV may be added as a pure compound, a solution of the pure compound in an inert solvent, or prepared in situ in the presence of the ester of Formula III. Many compounds of Formula IV are commercially available. Methods for preparing compounds of Formula IV, including in situ methods, from phosgene, phosgene equivalents such as diphosgene (trichloromethyl chloroformate)or triphosgene (bis(trichloromethyl)-carbonate), or thiophosgene and alcohols or phenols are known in the art. For compounds of Formula IV wherein W is O, see for example Carter, H. Z., Frank, R. L., Johnston, H. W. *Org. Syntheses.* **1943,** 23 and U.S. Patent 5,424,473. For compounds of Formula IV wherein W is S, see for example Martinez, M. A., Vega, J. C. *Synthesis*, **1986**(9), 760; McKay, A.F. *et al. Can J. Chem.* **1960,** 38 and FRG Patent 3616009. Reactions wherein HCl is liberated require a base to trap the acid. Suitable bases for this reaction include tertiary amines such trialkylamine or heteroarylamines such as imidazole, pyridine, picoline or other substituted pyridine, or mixtures thereof. The most preferred base is pyridine. Compounds of Formula IV may be isolated, such as by filtration or other suitable means, and purified, or treated in situ with compounds of Formula III. Base is also necessary to catalyze the combination of Formulae III and IV to yield compounds of Formula II. As previously stated, suitable base catalysts include trialkylamines, imidazole, pyridine, picolines or other substituted pyridines. After the formation of the compound of Formula II is complete, excess carbonylating agent can be decomposed by the addition of water. See McKenzie, A. and Lesslie, M. S., *Ber.,* **1928,** 61, 153-163 and Scott, J. W. and Parker, D., *Org. Prep.-Proced. Int.,* **1980,** 12, 242-3 for typical reaction conditions when Q and W are both O.

Alternatively, compounds of Formula IId (compounds of Formula II wherein Q is NH) can be prepared as in Scheme 3 by (1) treating compounds of Formula IIIc (compounds of Formula III wherein Q is NH) with phosgene (see for example Humphlett, W. J., Wilson, C. V., *J. Org. Chem.,* **1961,** 26 and Nowick, J. S. *et al. J. Org. Chem.,* **1992,** 57(26), 7364, Waldman, T. E., McGhee, W. D., *JCS Chem. Comm.* **1994,** 957 and Matthews, J., Rivero, R. A., *J. Org. Chem.,* **1997,** 62(17), 6090) or thiophosgene (see for example Nowick, J. S. *et al. J. Org. Chem.,* **1996,** 61(11), 3929) to prepare compounds of Formula V (that are racemic, enantiomerically enriched, or enantiomerically pure at chiral center *), or esters thereof, and (2) subsequently treating compounds of Formula V with compounds of Formula R³OH using literature procedures to form the corresponding (thiono)carbamates (Campaign, E. and Nargund, P. K., *J. Org. Chem.,* **1964,** *29*, 224; Skinner, G. S. and Vogt, H. C., *J. Amer. Chem. Soc.,* **1955,** *77*, 5440).

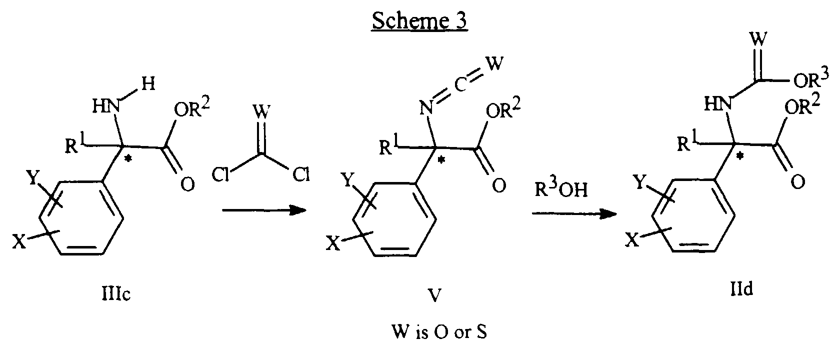

Compounds of Formula Va, or esters thereof, can also be prepared from compounds of Formula VI (that are racemic, enantiomerically enriched, or enantiomerically pure at chiral center *), or esters thereof, by treating with alkali metal or tetraalkylammonium salts of cyanate or thiocyanate (Scheme 4). The (thio)cyanate may also be contained in a solid phase, such as in polymeric ion-exchange resins.

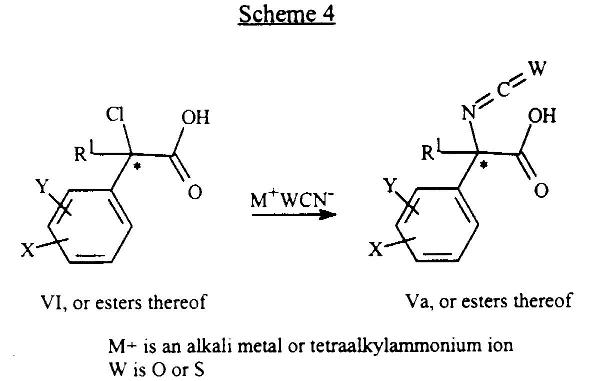

This method is similar to one described in the literature (Boehme, H., Martin, F. and Strahl, J., *Arch. Pharm.* **1980,** *313,* 10). An α-chloroester of Formula VI, prepared by literature methods (for a review, see Harwood, *Chem. Rev.,* **1962,** 62, 99-154, pp. 102-103; see also Schwenk, E. Papa, D., *J Am. Chem. Soc.,* **1948,** 70 and Robert, A., Jaguelin, S., Ounamant, J. L., *Tetrahedron,* **1986,** 42(8), 2275), is treated with a salt of a thiocyanate, such as potassium thiocyanate, in an inert solvent such as acetonitrile at a temperature from 0° to 50°C. When the reaction is complete, the reaction mixture is poured into water and extracted with a water-immiscible solvent such as chloroform. The organic extracts are combined,washed with water and the solvent is removed to give a residue which can be further purified or reacted with compounds of Formula R³OH as described in Scheme 3. (Thio)carbamates of Formula IId also can be prepared directly when treated with (thio)cyanate in the presence of a compound of Formula R³OH ( see Argabright, Rider and Sieck, *J. Org. Chem.,* **1965,** 30, 3317 and Effenberger, Drauz, Forster and Muller, *Chem. Ber.,* **1981,** 114, 173).

The 2-hydroxycarboxylic acids or esters of Formula III can be prepared by a number of methods known in the literature.
(1) The esters of Formula IIIb can be formed from the corresponding 2-hydroxycarboxylic acids by esterification as is well known in the literature. The 2-hydroxycarboxylic acids can be prepared from methyl ketones by formation of cyanohydrins, then hydrolysis, as is also known. For example, *Org. Syntheses.* Coll. Vol. 4, 58 (1968) teaches the preparation of atrolactic acid from acetophenone.
(2) The esters of Formula IIIb can also be synthesized from ketone cyanohydrins by treatment with alcohols in the presence of HCl to afford the iminoether hydrochlorides, followed by hydrolysis.
(3) A third method known for preparing 2-hydroxycarboxylic acids and esters involves treating 2-keto-acids or 2-keto-esters with nucleophilic-organometallic reagents such as Grignard reagents, and alkyl- and aryl-lithium reagents. For example, R. G. Salomon et al. teaches the preparation of some esters of Formula II by the addition of aryl-Grignard reagents to pyruvate esters *(J. Org. Chem.* (1982), *47,* 4692). Similarly, some 2-hydroxycarboxylic acids may be prepared by the regioselective nucleophilic addition of an aryl organometallic reagent to the metal salt (e.g., sodium salt) of pyruvic acid.
(4) Another method described in the literature for preparing some 2-aryl-2-hydroxyesters and acids is by acylation of aromatic rings with activated carbonyl compounds in the presence of a protic or Lewis acid. Aromatic substrates capable of undergoing reactions of this type are benzene, diphenyl ether, and other aromatic compounds known to be of sufficient reactivity to undergo Friedel-Crafts-type reactions. In the case of mono-substituted benzene derivatives, the acylation occurs preferentially, but not necessarily exclusively, para to the point of attachment of the substituent. For example, see *Org. Syntheses,* Coll. Vol. *3,* 326, (1955); Salomon et al., *J. Org. Chem.,* (1982), *47*, 4692; and U.S. 4,922,010. Carbonyl compounds known to undergo this acylation reaction include pyruvate esters and acids, glyoxylate esters and acids, and diesters of oxomalonates. The acids used in the acylation reaction can either be protic in nature, for example, a mixture of acetic and sulfuric acid, or a Lewis acid such as aluminum chloride, tin tetrachloride, titanium tetrachloride, or other Lewis acid known to effect Friedel-Crafts-type reactions. The acid can be used either catalytically or in excess. In some cases, the acid may react destructively with the carbonyl substrate and excess carbonyl compound must be used.
(5) Another method described in the literature for preparing some 2-aryl-2-hydroxyesters and acids is by electrochemical carboxylation of aryl methyl ketones. See U. S. Patents 4,582,577 and 4,601,797.

The esters of a-amino-acids of Formula IIId (compounds of Formula III wherein Q is NH) or their salts are known in the literature and can be prepared by literature methods. For a review, see J. P. Greenstein, M. Winitz, "Chemistry of the Amino Acids" (S. Patai, Ed.), p 697, John Wiley and Sons, Ltd., London (1961). See also Charette, A. B. and Mellon, C. *Tetrahedron,* **1998,** 54, 10525-10535; Matsushita, M. Maeda, H. and Kodama, M., *Tetrahedron Letters,* **1998,** 39, 3749-3752; Obrecht, D., *et al., Helv. Chim. Acta,* 1995, 78, 563-580; Mitani, M. *et al., Chem. Letters,* **1987,** 1029-1030; and Loev, B., Macko, E. and Fried, I. *M., J Med. Chem.,* **1969,** 12, 854-9 for preparation of some compounds of Formula IId. The group R² in compounds of Formula IId can be C₁-C₄ alkyl; C₁-C₄ haloalkyl; or phenyl, benzyl or phenethyl, each optionally substituted on the aromatic ring with halogen, nitro, C₁-C₄ alkyl, C₁-C₄ haloalkyl, C₁-C₄ alkoxy, C₁-C₄ haloalkoxy and/or cyano. Preferred for ease of synthesis and lower expense are those compounds wherein R² is C₁-C₄ alkyl. The preparation of the isothiocyanates of Formula V from the α-amino esters or their salts of Formula III can also be accomplished by literature methods (Staab, H. A. and Walther, *G., Liebigs Ann. Chem.,* **1962,** *657,* 98; Moore, M. L. and Crossley, F. S., *Org. Synth.,* **1941,** *21,* 81).

Without further elaboration, it is believed that one skilled in the art using the preceding description can utilize the present invention to its fullest extent. The following Examples are, therefore, to be construed as merely illustrative, and not limiting of the disclosure in any way whatsoever. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; s = singlet, d = doublet, t = triplet, q = quartet, m = multiplet, dd = doublet of doublets, dt = doublet of triplets, br s = broad singlet. The following abbreviations are used in the Examples below: TLC is Thin Layer Chromatography and HPLC is High-Pressure Liquid Chromatography.

### Example 1

### Preparation of Ethyl 2-Phenoxycarbonyloxy-2-(4-phenoxyphenyl)propionate

To a suspension of ethyl 2-hydroxy-2-(4-phenoxyphenyl)propionate (22 g, 76.83 mmol) in heptane (37 ml) was added dry pyridine (6.84 mL, 84.51 mmol) and then dropwise phenyl chloroformate (10.60 mL, 84.51 mmol) at room temperature. After the addition, the mixture was stirred for 10 h at 90 °C. The reaction was monitored by TLC (20% EtOAc/hexane) or alternatively by HPLC.† The solid was filtered and washed with water to dissolve the pyridinium salt. Sometimes if the ethyl 2-hydroxy-2-(4-phenoxyphenyl)propionate was too impure, the mixture was sonicated in heptane (30 min) before filtering the solid. The white solid, m.p. 65-66 °C., ethyl 2-phenoxycarbonyloxy-2-(4-phenoxyphenyl)propionate, was obtained with practically quantitative yield. The compound was completely identified by ¹H NMR (CDCl₃): δ 1.29 (t, J= 7.2Hz, 3H), 2.13 (s, 3H), 4.27 (m, 2H), and 7.00-7.60 (m, 14H). ¹³C NMR (CDCl₃): δ 13.9, 23.4, 62.0, 84.0, 118.3, 119.2, 120.9, 123.6, 126.1, 126.5, 129.4, 129.7, 133.2, 150.9, 151.9, 156.4, 157.5, and 170.0.

This procedure was also carried out with other solvents such as xylene and methylene chloride (at around 40 °C) obtaining results similar to as those described above.

### Example 2

### Preparation of Methyl 2-Phenoxycarbonyloxy-2-14-phenoxyphenyl)propionate

To a suspension of methyl 2-hydroxy-2-(4-phenoxyphenyl)propionate (10 g, 36.72 mmol) in heptane (14 mL) was added dry pyridine (3.27 mL, 40.39 mmol) and then dropwise phenyl chloroformate (5.07 mL, 40.39 mmol) at room temperature. After the addition, the mixture was stirred for 8 h at 90 °C. The reaction was monitored by TLC (20% EtOAc/hexane) or alternatively by HPLC.† The solid was filtered and washed with water to dissolve the pyridinium salt. The white solid, , m.p. 95-97 °C, methyl 2-phenoxycarbonyloxy-2-(4-phenoxyphenyl)propionate, was obtained with practically quantitative yield. The compound was completely identified by ¹H NMR (CDCl₃): δ 2.14 (s, 3H), 3.80 (s, 3H) and 7.00-7.60 (m, 14H). ¹³C NMR (CDCl₃): δ 23.4, 52.9, 83.9, 118.3, 119.2, 120.9, 123.6, 126.1, 126.5, 129.4, 129.7, 133.1, 150.8, 151.9, 156.4, 157.6 and 170.6.

### Example 3

### Preparation of Ethyl 2-(p-Nitrophenoxycarbonyloxyl-2-(4-phenoxyphenyl)propionate

To a suspension of ethyl 2-hydroxy-2-(4-phenoxyphenyl)propionate (10 g, 34.92 mmol) in heptane (14 mL) was added dry pyridine (3.27 mL, 40.39 mmol) and then dropwise *p*-nitrophenyl chloroformate (8.14 g, 40.39 mmol) at room temperature. After the addition, the mixture was stirred for 8 h at 90 °C. The reaction was monitored by TLC (20% EtOAc/hexane) or alternatively by HPLC.† The solid was filtered and washed with water to dissolve the pyridinium salt. The white solid, m.p. 130-131 °C, ethyl 2-(*p*-nitrophenoxycarbonyloxy)-2-(4-phenoxyphenyl)propionate, was obtained with practically quantitative yield. The compound was completely identified by ¹H NMR (CDCl₃): δ 1.28 (t, J= 7.2Hz, 3H), 2.13 (s, 3H), 4.27, (m, 2H), 6.99-7.52 (m, 11H) and 8.32 (d, J = 9.2 Hz, 2H). ¹³C NMR (CDCl₃): δ 13.9, 23.2, 62.2, 84.8, 118.3, 119.3, 121.7, 123.8, 125.3, 126.6, 129.8, 132.6, 145.4, 150.7, 155.3, 156.3, 157.8, and 169.6.

### Example 4

### Preparation of Methyl 2-(p-Nidophenoxycarbonyloxy)-2-(14-phenoxyphenyl)propionate

To a suspension of methyl 2-hydroxy-2-(4-phenoxyphenyl)propionate (10 g, 36.72 mmol) in heptane (14 mL) was added dry pyridine (3.27 mL, 40.39 mmol) and then dropwise p-nitrophenyl chloroformate (8.14 g, 40.39 mmol) at room temperature. After the addition, the mixture was stirred for 8 h at 90 °C. The reaction was monitored by TLC (20% EtOAc/hexane) or alternatively by HPLC.† The solid was filtered and washed with water to dissolve the pyridinium salt. The white solid, m.p. 106-108 °C, methyl 2-*(p*-nitrophenoxycarbonyloxy)-2-(4-phenoxyphenyl)propionate, was obtained with practically quantitative yield. The compound was completely identified by ¹H NMR (CDCl₃): δ 2.14 (s, 3H), 3.81, (s, 3H), 7.00-7.62 (m, 11H) and 8.30 (d, J = 9.2 Hz, 2H). ¹³C NMR (CDCl₃): δ 23.1, 53.0, 84.7, 118.2, 119.3, 121.6, 123.8, 125.2, 126.5, 129.8, 132.4, 145.3, 150.7, 155.2, 156.3, 157.9 and 170.2.

### Example 5

### Preparation of Ethyl alpha-[(ethoxycarbonyl)oxy]-alpha-methyl-4-phenoxybenzeneacetate

Ethyl 2-(4-phenoxyphenyl)lactate (1 g, 3.5 mmol) was dissolved in tetrahydrofuran (20 mL) and cooled to 0°C. Sodium hydride (97%, 92 mg, 3.8 mmol) was added as a solid in one portion. Gas evolution ensued. The reaction mixture was warmed to room temperature, stirred 15 minutes, and then recooled to 0°C. Ethyl chloroformate (0.37 mL, 3.8 mmol) was then added. Thin layer chromatography showed lactate still present after 15 minutes. The reaction was warmed to room temperature and stirred an additional 30 minutes. Additional ethyl chloroformate was added (0.35 mL) and the reaction mixture was stirred for approximately 60 hours. The reaction mixture was re-cooled to 0oC and more sodium hydride (approximately 50 mg) was added. The reaction mixture was warmed to room temperature and more ethyl chloroformate (0.37 mL) was added . After stirring for 3 h, the reaction mixture was poured into aqueous 1 N HCl and extracted two times with diethyl ether (50 mL each). The ether phases were separated and each washed with water (50 mL) and then brine (50 mL). The combined organic phases were then dried (MgSO₄). The organic phases were evaporated under reduced pressure to give a light yellow oil (1.18 g). ¹H NMR (90 MHz, CDCl₃) suggested approximately a 50:50 mixture of starting lactate and desired product as evidenced by the 2 methyl singlets at 2.5 and 2.3 ppm. 8.2-7.4 (m, 18H, 2-ArH); 4.9-4.5(m,6H, 3-OCH₂); 2.5 (s,3H, CH₃), 2.3 (s,3H, CH₃); 2.0-1.6 (m,9H, 3-CH₃).

### Example 6

### Preparation of Methyl alpha-methyl-alpha-[(phenoxycarbonyl)oxy]benzeneacetate

### Methyl 2-phenyllactate (2.00 g, 11.1 mmol) was dissolved in diethyl ether (30 mL) and cooled to 0°C. Potassium tert-butoxide (1.37 g, 12.2 mmol) was then added. The reaction mixture was warmed to room temperature, stirred for 30 min, and then re-cooled to 0°C. Phenyl chloroformate (1.53 mL, 12.2 mmol) was then added and the reaction mixture was immediately warmed to room temperature. Thin layer chromatography showed the lactate consumed after 15 min. The reaction mixture was poured into water (80 mL) and extracted two times with diethyl ether (80 mL each). The organic extracts were each washed with water (80 mL) and brine (80 mL). The combined organic phases were combined and dried (MgSO₄). The solvent, unreacted phenyl chloroformate, and a small amount of unreacted methyl 2-phenyllactate were removed under reduced pressure. The residue (2.52 g) was purified by column chromatography on silica (eluant = 1:10 v/v ethyl acetate:hexanes) to give clean product (1.24 g). ¹H NMR (200 MHz, CDCl₃): 7.6-7.2 (m,10H); 3.8 (s,3H); 2.1 (s,3H).

### Example 7

### Preparation of 5-Methyl-5-(4-phenoxyphenyl)-3-(phenylamino)-2,4-oxazolidinedione

To a solution of ethyl 2-phenoxycarbonyloxy-2-(4-phenoxyphenyl)propionate (6 g, 14.76 mmol) and 4-dimethylaminopyridine (0.18 g, 1.48 mmol) in 1,4-dioxane (24 mL) was added, under nitrogen atmosphere, acetic acid (0.84 mL, 14.76 mmol) and phenyl hydrazine (2.90 ml, 29.52 mmol). The mixture was stirred at 80 °C for 12 h, until no starting material was detected by TLC (20% EtOAc/hexane) or alternatively by HPLC.† After cooling, the solution was evaporated to dryness, the residue was dissolved in methylene chloride (20 mL), water was added (20 mL), and then the solution was extracted with methylene chloride (3 x 20 mL). The combined organic layers was dried (Na₂SO₄) and evaporated to give a solid, which was washed with heptane to afford 4.59 g (83%) of 5-methyl-5-(4-phenoxyphenyl)-3-(phenylamino)-2,4-oxazolidinedione, m.p. 148-150 °C. The compound was completely identified by ¹H NMR (CDCl₃): δ 2.00 (s, 3H, ), 6.23 (br s, 1H, ), and 6.80-7.60 (m, 14H,). ¹³C NMR (CDCl₃): δ 25.3, 85.0, 114.0, 118.5, 119.4, 123.0, 124.0, 126.0, 129.4, 129.8, 130.1, 144.1, 152.9, 156.1, 158.4, and 172.0.

This procedure was also carried out with acetonitrile (at about 81 °C), tetrahydrofuran (at about 68 °C), and anisole (at about 90 °C) as solvents obtaining results similar to as those described above except that product formation was followed by HPLC by looking for the appearance of 5-methyl-5-phenoxyphenyl-3-(phenylamino)-2,4-oxazolidinedione and the disappearance of the starting material ethyl 2-phenoxycarbonyloxy-2-(4-phenoxyphenyl)pronionate.

† HPLC Method: Column: 25cm x 4.6 mm inner diameter Zorbax@RX-C8 (5
µm particles) [Part # 880967.901 MAC-MOD Analytical, Inc., Chadds Ford, PA]; flow:
1.5 ml / min; column temperature: 40 °C; injection volume: 10 ml; detector
wavelength: 230 nm (bandwidth 4 nm); reference wavelength: 400 nm (bandwidth 80
nm) and solvent gradient:

| Time(min) | MeCN (%) | H₂O-H₃PO₄ Ph= 3 solution (%) |
|---|---|---|
| 0.0 | 40 | 60 |
| 45.0 | 85 | 15 |
| 45.1 | 40 | 60 |

| Approximate Retention times: | |
|---|---|
| Ethyl 2-hydroxy-2-(4-phenoxyphenyl)propionate | 11.3 min. |
| Methyl 2-hydroxy-2-(4-phenoxyphenyl)propionate | 8.8 min. |
| Ethyl 2-phenoxycarbonyloxy-2-(4-phenoxyphenyl)propionate | 25.9 min. |
| Ethyl 2-(*p*-nitrophenoxycarbonyloxy)-2-(4-phenoxyphenyl)propionate | 25.5 min. |
| Methyl 2-phenoxycarbonyloxy-2-(4-phenoxyphenyl)propionate | 23.7 min. |
| 5-Methyl-5-(4-phenoxyphenyl)-3-(phenylamino)-2,4-oxazolidinedione | 17.5 min. |

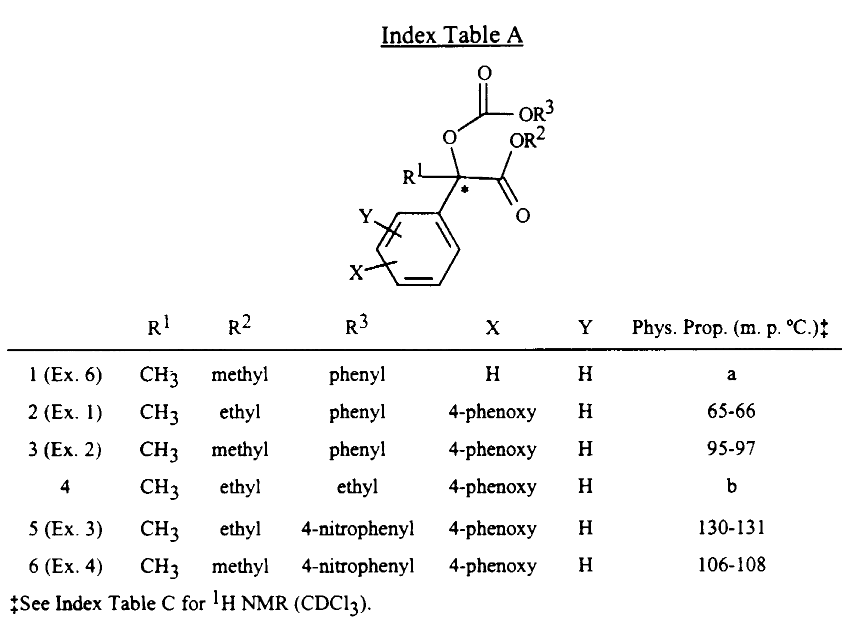

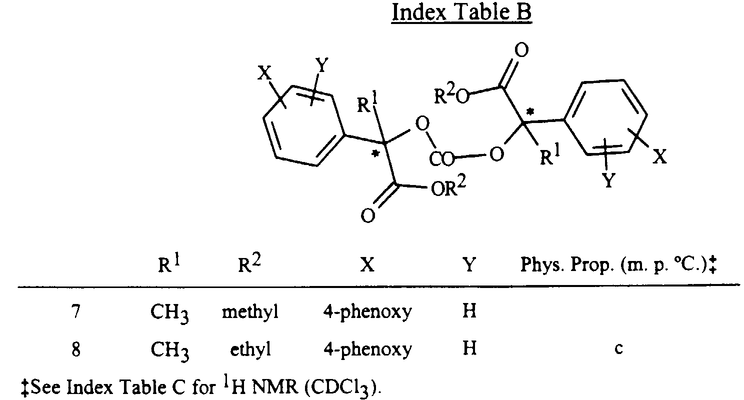

| Index Table C (¹H NMR in CDCl₃ (δ).) |
|---|
| a) 2.11 (s,3H), 3.76 (s,3H), 7.2-7.6 (m,10H). |
| b) 1.23 (t,3H), 1.35 (t,3H), 2.01 (s,3H), 4.2 (m,4H). 6.9-7.5 (m,9H). |
| c) 1.22 (t,6H), 2.02 (d,6H), 4.08-4.30 (m,4H), 6.96-7.02 (m,8H), 7.10 (m,2H), 7.34 (m,4H), 7.52 (m,4H). |

Compounds of Formula II may exhibit fungicidal activity. For example, Compound 1 (see Index Table A) has activity against Grape Downy Mildew. However, as discussed above, in accordance with this invention, they are considered to be important intermediates for preparing compounds of Formula I.

## Claims

1. A process for preparing a compound of Formula I 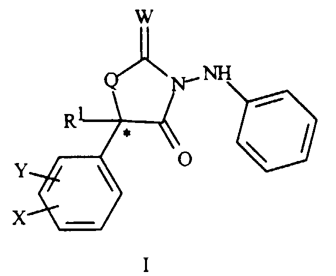 wherein Q is O or NH; W is O or S; X is H, halogen, C₁-C₄ alkyl, C₁-C₄ haloalkyl or OR⁴; Y is H or halogen; R¹ is C₁-C₄ alkyl; R⁴ is C₁-C₄ alkyl, C₁-C₄ haloalkyl or optionally substituted phenyl; and * represents the location of the chiral center; comprising:
treating a compound of Formula II, or an ester therof, 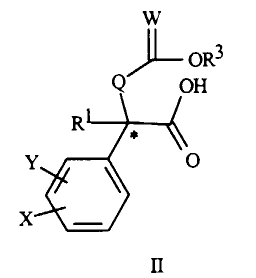 wherein OR³ is an oxygen-linked coupling moiety, with phenyl hydrazine in the presence of an acidic catalyst.

2. The process of Claim 1 for preparing a compound of Formula I that is enriched in the stereoisomer Ic 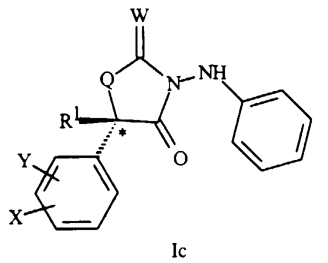 wherein a compound of Formula II that is enriched in the stereoisomer of Formula IIc, or an ester thereof, 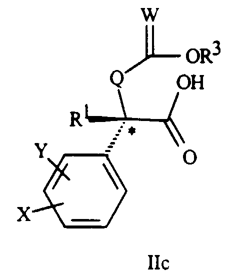 is treated with phenyl hydrazine in the presence of an acidic catalyst.

3. The process of Claim 1 wherein a basic catalyst is also present.

4. The process of Claim 3 wherein the acidic catalyst is selected from carboxylic acids, Lewis acids, and pyridine carboxylic acids; and the basic catalyst is selected from tertiary amines and heteroaryl amines.

5. The process of Claim 3 wherein the compound of Formula II is an ester of Formula IIa 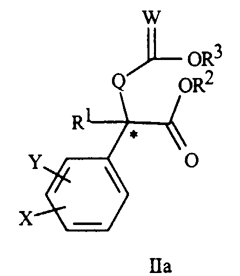 wherein
R² is C₁-C₄ alkyl; C₁-C₄ haloalkyl; or phenyl, benzyl or phenethyl, each optionally substituted on the aromatic ring with one or more substituents selected from halogen, nitro, C₁-C₄ alkyl, C₁-C₄ haloalkyl, C₁-C₄ alkoxy, C₁-C₄ haloalkoxy and cyano; and
R³ is phenyl optionally substituted with one or more substituents selected from halogen, nitro, C₁-C₄ alkyl, C₁-C₄ haloalkyl, C₁-C₄ alkoxy, C₁-C₄ haloalkoxy and cyano;
is treated with phenyl hydrazine; the acid catalyst is selected from the group consisting of acetic acid, pivalic acid, benzoic acid and nicotinic acid; the basic catalyst is *N,N*-dimethylaminopyridine; the reaction is carried out in a solvent selected from the group anisole, xylene, tetrahydrofuran, 1,4-dioxane or acetonitrile; and the temperature is in the range from 50 to 100° C.

6. The process of Claim 1 wherein Q is O; W is O; X is H, halogen or 4-phenoxy; Y is H; and R¹ is methyl.

7. The process of Claim 6 wherein the compound of Formula II is prepared by treating a compound of Formula IIIa, or an ester thereof, 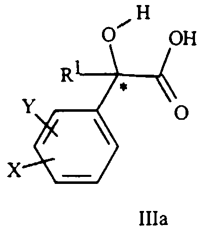 with a chloroformate of Formula IVa 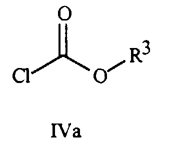 wherein R³ is C₁-C₄ alkyl or phenyl optionally substituted with one or more substituents selected from halogen, nitro, C₁-C₄ alkyl, C₁-C₄ haloalkyl, C₁-C₄ alkoxy, C₁-C₄ haloalkoxy and cyano; in the presence of a base in an inert solvent at a temperature from 0 to 150° C.

8. The process of Claim 1 wherein Q is NH; W is S; X is H, halogen, C₁-C₄ alkyl, C₁-C₄ haloalkyl or OR⁴; Y is H or halogen; R¹ is methyl; and R⁴ is C₁-C₄ alkyl, C₁-C₄ haloalkyl or optionally substituted phenyl.

9. A compound of Formula II or an ester thereof, useful as an intermediate to fungicides 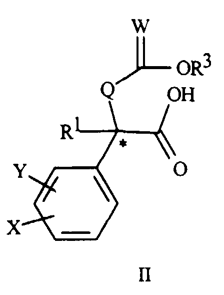 wherein Q is O or NH; W is O or S; X is H, halogen, C₁-C₄ alkyl, C₁-C₄ haloalkyl or OR⁴; Y is H or halogen; R¹ is C₁-C₄ alkyl; R³ is an oxygen-linked coupling moiety; and R⁴ is C₁-C₄ alkyl, C₁-C₄ haloalkyl or optionally substituted phenyl; provided that (a) when Q and W are both O and X and Y are both H, then OR³ is other than CH₃; and (b) when Q is NH, then (i) R³ is other than ethyl, benzyl and benzyl substituted with -N=N-phenyl, and (ii) the esters of Formula II are other than C₁-C₄ alkyl esters, C₃-C₄ alkenyl esters, C₃-C₆ cycloalkyl esters and benzyl esters.

10. The compound of Claim 9 that is enriched in the stereoisomer IIc, or an ester thereof

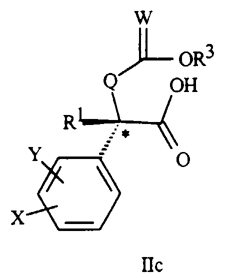

11. The compound of Claim 9, of Formula IIa 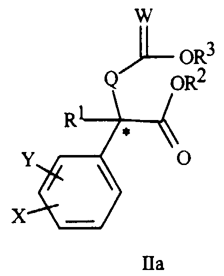 wherein
R² is C₁-C₄ alkyl; C₁-C₄ haloalkyl; or phenyl, benzyl or phenethyl, each optionally substituted on the aromatic ring with one or more substituents selected from halogen, nitro, C₁-C₄ alkyl, C₁-C₄ haloalkyl, C₁-C₄ alkoxy, C₁-C₄ haloalkoxy and cyano; and
R³ is a phenyl optionally substituted with one or more substituents selected from halogen, nitro, C₁-C₄ alkyl, C₁-C₄ haloalkyl, C₁-C₄ alkoxy, C₁-C₄ haloalkoxy and cyano.
12. The compound of Claim 11 wherein Q is O; W is O; X is H or 4-phenoxy; Y is H; R¹ is methyl; R² is C₁-C₂ alkyl; and R³ is phenyl.
